# EUROPEAN PATENT APPLICATION

(11) **EP 4 775 561 A1**
(43) Date of publication of application: **15.07.2026**
(21) Application number: 25150843.8
(22) Date of filing: 09.01.2025
(51) Int. Cl.: C07C 29/10, C07C 31/20

(54) **METHOD FOR PRODUCING ALKYLENE GLYCOL**

(71) Applicant: SABIC Global Technologies B.V., 4612 PX Bergen op Zoom (NL)
(72) Inventor: Jain, Jai Prakash, 6160 GA Geleen (NL); Gopal, Srikant, 6160 GA Geleen (NL)
(74) Representative: Sabic INDIA Intellectual Property Group

(57) **Abstract**

The invention relates to a method for producing monoalkylene glycol, the method comprising:
a) flowing a feed stream comprising alkylene oxide and water into a first reactor unit;
b) subjecting the feed stream, in the first reactor unit, to first reaction conditions to obtain a first effluent comprising monoalkylene glycol, unreacted alkylene oxide and unreacted water,
wherein the first reaction conditions in the first reactor unit are non-catalytic reaction conditions;
c) flowing at least a portion of the first effluent to a second reactor unit; and
d) subjecting said portion of the first effluent, in the second reactor unit, to second reaction conditions to obtain a second effluent comprising monoalkylene glycol, wherein the second reactor unit comprises a reactor comprising a catalyst bed and the second reaction conditions in the second reactor unit are catalytic reaction conditions.

## Description

The present invention relates to a method for producing alkylene glycol.

Alkylene glycols are very useful compounds. For example, monoethylene glycol (MEG) is used as an antifreeze and coolant for engines and intermediate for producing polyester fibers and polyethylene terephthalate (PET), which is used for producing plastic bottles. Diethylene glycol (DEG) can be used to produce polyurethanes, plasticizers, and organic solvents. Triethylene glycols (TEG) are often used as plasticizers and moistureretaining agents. Polyethylene glycols (PEG) are used in perfumes, cosmetics, lubricants, and plasticizers.

The conventional process for producing ethylene glycols reacts ethylene oxide (EO) with water (H₂O) to produce Monoethylene glycol (MEG), diethylene glycol (DEG) and triethylene glycol (TEG). The reaction of EO with water is a thermal non-catalytic liquid phase reaction. The MEG produced further reacts with EO to form DEG, which can again react with EO to produce TEG. A large excess of water is used in the reaction. Typically in such process, the molar ratio of H₂O/EO ranges from 20-26 and yields 90-91% MEG, ~9% DEG, and 0.5-1% TEG.

In some instances, MEG is desired more than DEG and TEG. The MEG selectivity can be increased by increasing water/EO ratio, but there is a limit to the achievable MEG selectivity in a conventional plant. It is also highly desirable to reduce the water/EO ratio to reduce the energy requirement in the process.

WO2022079662A1 discloses a method of producing alkylene glycol by a combination of a plug flow reactor and a second reactor comprising a reactive distillation column operating under non-catalytic conditions.

WO1998014419A1 discloses a method for making glycol in a multiple effect evaporation column reactor. Each of the reactors can have a catalyst bed. Each subsequent evaporation column effect in series can have a reaction zone of higher catalytic activity than the previous effect in series.

EP3181542A1 discloses a method for preparing an alkylene glycol comprising hydrating with water an alkylene oxide in a hydrating reactor placed in a reaction zone. In the hydrating reactor, the alkylene oxide reacts with water in a catalytic or non-catalytic process without use of other intermediates such as carbonates. Non-catalytic process is preferred.

It is an objective of the present invention to provide a method by which the above-mentioned and/or other needs are met.

Accordingly, the present invention provides a method for producing monoalkylene glycol, the method comprising:
a) flowing a feed stream comprising alkylene oxide and water into a first reactor unit;
b) subjecting the feed stream, in the first reactor unit, to first reaction conditions to obtain a first effluent comprising monoalkylene glycol, unreacted alkylene oxide and unreacted water,
   wherein the first reaction conditions in the first reactor unit are non-catalytic reaction conditions;
c) flowing at least a portion of the first effluent to a second reactor unit; and
d) subjecting said portion of the first effluent, in the second reactor unit, to second reaction conditions to obtain a second effluent comprising monoalkylene glycol, wherein the second reactor unit comprises a reactor comprising a catalyst bed and the second reaction conditions in the second reactor unit are catalytic reaction conditions.

The method according to the invention requires a first reactor unit operating under non-catalytic conditions (non-catalytic reactor) followed by a second reactor unit operating under catalytic conditions (catalytic reactor). The use of a non-catalytic reactor is advantageous for a simple construction of the system with lower capital expenditures and operating expenses and the use of a following catalytic reactor leads to a high selectivity towards monoalkylene glycol at a low water/alkylene oxide ratio, allowing substantial reduction of energy consumption.

The invention provides a method for producing monoalkylene glycol. Preferably, the monoalkylene glycol is monoethylene glycol and the alkylene oxide is ethylene oxide. Accordingly, in the present description, the mention of monoalkylene glycol and alkylene oxide is understood to mention also as monoethylene glycol and ethylene oxide, respectively.

### Step a)

The method comprises flowing a feed stream comprising alkylene oxide and water into a first reactor unit. The feed stream may consist of alkylene oxide and water. The feed stream may further comprise monoalkylene glycol, but preferably does not comprise monoalkylene glycol. Alkylene oxide and water may be fed to the first reactor unit together or separately.

The molar ratio between the water and the alkylene oxide in the feed stream may e.g. be 5:1 to 30:1, preferably 6:1 to 25:1, more preferably 7:1 to 20:1, more preferably 8:1 to 18:1, more preferably 9:1 to 15:1. A low molar ratio between the water and the alkylene oxide allows reducing energy consumption. According to the invention, a high selectivity towards monoalkylene glycol is achieved even at a low water/alkylene oxide ratio, allowing substantial reduction of energy consumption.

### Step b)

The method comprises subjecting the feed stream, in the first reactor unit, to first reaction conditions to obtain a first effluent comprising monoalkylene glycol, unreacted alkylene oxide and unreacted water.

The first reactor conditions are selected such that alkylene oxide and water react to form monoalkylene glycol. Dialkylene glycol and/or trialkylene glycol may also be formed. Not all alkylene oxide will react with water in the first reactor unit. Accordingly, the first effluent obtained by the first reactor unit comprises monoalkylene glycol, unreacted alkylene oxide and unreacted water and may further comprise dialkylene glycol and/or trialkylene glycol and/or polyalkylene oxide. The first reactor unit is configured to release the first effluent therefrom.

The first reaction conditions in the first reactor unit are non-catalytic reaction conditions. Thus, the first reactor unit comprises substantially no catalyst or comprises no catalyst.

Preferably, the first reactor unit comprises a plug flow reactor adapted to provide the first reaction conditions therein.

The first reactor unit may may comprise a heating mechanism adapted to heat water and alkylene oxide to a reaction temperature sufficient to produce monoalkylene glycol. The heating mechanism may include a heat exchanger located upstream to an inlet of the first reactor unit.

The first reaction conditions in the first reactor unit may comprise a temperature in a range of 60 to 200 °C, for example 70 to 190 °C, and a pressure of 10 to 30 bar, for example 15 to 25 bar.

The first reactor unit may have a reaction residence time of 0.1 to 8 minutes, e.g. 1.0 to 6 minutes.

As mentioned above, reaction of alkylene oxide with water is not complete in the first reactor unit. For example, the amount of alkylene oxide in the first effluent with respect to the amount of alkylene oxide in the feed stream fed to the first reactor may be 20 to 80 wt%.

The first effluent comprises some amount of unreacted alkylene oxide. The amount of alkylene oxide in the first effluent with respect to the total weight of the first effluent may e.g. be 1.0 to 20 wt% or 3.0 to 15 wt%.

The amount of monoalkylene glycol in the first effluent with respect to the total weight of the first effluent may e.g. be 1.0 to 25 wt% or 2.0 to 20 wt%.

The amount of alkylene oxide in the first effluent with respect to the amount of monoalkylene glycol in the first effluent may e.g. be 50 to 150 wt%, for example 50 to 100 wt% or more than 100 wt% and at most 150 wt%.

The reaction conditions are selected to allow a higher selectivity towards monoalkylene glycol than dialkylene glycol and trialkylene glycol. Preferably, the amount of monoalkylene glycol in the first effluent with respect to the amount of monoalkylene glycol, dialkylene glycol and trialkylene glycol in the first effluent is at least 85.0 wt%, more preferably at least 90.0 wt%, more preferably 91.5 wt%, preferably at least 92.0 wt%, more preferably at least 92.5 wt%, more preferably at least 93.0 wt%, more preferably at least 93.5 wt%, more preferably at least 94.0 wt%, more preferably at least 94.5 wt%, more preferably at least 96 wt%, more preferably at least 98 wt%.

### Step c)

The method comprises flowing at least a portion of the first effluent to a second reactor unit. The first reactor unit may comprise an outlet which is in fluid communication with an inlet of the second reactor unit such that the first effluent flows from the first reactor unit to the second reactor unit.

Preferably, the method comprises flowing substantially all of the first effluent or flowing all of the first effluent to a second reactor unit, e.g. the method comprises flowing at least 90 wt%, at least 98 wt%, at least 99 wt% or 100 wt% of the first effluent to a second reactor unit.

### Step d)

The method comprises subjecting said portion of the first effluent, in the second reactor unit, to second reaction conditions to obtain a second effluent comprising monoalkylene glycol. The second reactor unit comprises a reactor comprising a catalyst bed. The second reaction conditions in the second reactor unit are catalytic reaction conditions.

In some preferred embodiments, the reactor comprising the catalyst bed is a reactive distillation column. This result in a particularly high selectivity towards monoalkylene glycol.

In some preferred embodiments, the reactor comprising the catalyst bed is a catalytic fixed bed reactor.

The second reactor conditions are catalytic reaction conditions selected such that unreacted alkylene oxide and unreacted water in said portion of the first effluent react to form monoalkylene glycol. Thus, the amount of monoalkylene glycol in the second effluent is higher than the amount of monoalkylene glycol in said portion of the first effluent fed to the second reactor unit.

Preferably, substantially all or all of the unreacted alkylene oxide in said portion of the first effluent is converted into monoalkylene glycol. Preferably, the amount of alkylene oxide in the second effluent with respect to the amount of alkylene oxide in said portion of the first effluent fed to the second reactor is at most 10 wt%, at most 5.0 wt%, at most 2.0 wt%, at most 1.0 wt%, at most 0.5 wt%, at least 0.1 wt% or 0.0 wt%.

Preferably, the second effluent comprises substantially no alkylene oxide or comprises no alkylene oxide. Preferably, the amount of alkylene oxide in the second effluent with respect to the total weight of the second effluent is at most 2.0 wt%, at most 1.0 wt%, at most 0.5 wt%, at least 0.1 wt% or 0.0 wt%.

Preferably, the amount of monoalkylene glycol in the second effluent with respect to the total weight of the second effluent is 10 to 25 wt%.

Preferably, the amount of alkylene oxide in the second effluent with respect to the amount of monoalkylene glycol in the second effluent is at most 10 wt%, at most 5.0 wt%, at most 2.0 wt%, at most 1.0 wt%, at most 0.5 wt%, at least 0.1 wt% or 0.0 wt%.

The catalyst reaction conditions are selected to allow a higher selectivity towards monoalkylene glycol than dialkylene glycol and trialkylene glycol. Accordingly, preferably, the amount of monoalkylene glycol in the second effluent with respect to the total amount of monoalkylene glycol, dialkylene glycol and trialkylene glycol in the second effluent is at least 80.0 wt%, preferably at least 85.0 wt%, more preferably at least 90.0 wt%, more preferably 91.5 wt%, more preferably at least 92.0 wt%, more preferably at least 92.5 wt%, more preferably at least 93.0 wt%, more preferably at least 93.5 wt%, more preferably at least 94.0 wt%, more preferably at least 94.5 wt%.

The catalyst bed comprises a catalyst. The catalyst may be selected from known acid catalysts described in WO1998/14419: fluoriated alkyl sulfonic acid ion exchange resins (US Pat No 4,165,440), carboxylic acids and halogen acids (US Pat No 4,112,054), strong acid cation exchange resins (US Pat No 4,107,221), aliphatic mono- and/or polycarboxyhc acids (US Pat No 3,933,923), cationic exchange resins (US Pat No 3,062,889), acidic zeolites (US Pat No 3,028,434), sulfur dioxide (US Pat No 2,807,651), trihalogen acetic acids (US Pat No 2,472,417), and copper-promoted aluminum phosphate (US Pat No 4,014,945). The catalyst may be selected from heterogeneous catalysts such as aluminosilicate zeolites, amorphous aluminosilicates, and acid form ion exchange resins. A preferred catalyst is a perfluorosulfonic acid resin entrapped within and dispersed throughout a carrier of metal oxide. Preferably, the metal oxide is selected from the group consisting essentially of silica, alumina, titania, germania, zirconia, alumino-silicate, zirconyl-silicate, chromic oxide and iron oxide. Such types of catalysts are described in US 4,731,263 and WO1995/19222.

The catalyst may be selected from the group consisting of
molecular sieves such as zeolites,
metal oxides and supported metal oxides,
metals and supported metals,
niobic acid,
hydrotalcite and hydrotalcite modified with metals,
heteropolyacids,
ion-exchange resins,
heterogenized metal complexes, such as Co/Salen on silica or polymer supports,
solid superacids or superbases, and
mesoporous molecular sieves in acidic or basic form with supported or exchanged metals
and combinations thereof.

The zeolites may e.g. be crystalline aluminosilicate zeolites having a pore size of 4-13 angstrom; alkali metal and alkaline earth metal aluminosilicates (such as sodium aluminosilicates and calcium aluminosilicates) or zeolites modified with metals such as Sn or Cs.

The metal oxides and supported metal oxides may e.g. be niobium oxide and supported niobium oxide.

The supported metals may e.g. be example metals belonging to Group 1 or 2 on a support or a supported niobium.

In addition to the second effluent, an overhead stream comprising unreacted water and unreacted alkylene oxide may be obtained from the second reactor unit. In this case, the second effluent is provided as a bottoms stream comprising unreacted water and monoalkylene glycol. Preferably, at least a portion of the overhead stream is recycled back to the reactor comprising the catalyst bed as reflux. Preferably, the bottoms stream is heated by a reboiler and primarily alkylene oxide is recycled back to the reactor comprising the catalyst bed, resulting in the second effluent with a low amount of alkylene oxide.

The overhead stream can be recycled back to the first reactor unit for producing additional alkylene glycol or can be sent for pure alkylene oxide production.

The overhead stream may comprise e.g. 0 to 50 wt% of unreacted alkylene oxide and 50 to 100 wt% of unreacted water.

Preferably, the second reaction conditions in the second reactor unit comprise a reactor inlet temperature in a range of 80 to 220 °C, for example 160 to 220 °C, and a reaction pressure of 10 to 30 bar, for example 10 to 20 bar.

In some embodiments, the method involves using only the first reactor unit and the second reactor unit as reactor units for converting alkylene oxide and water to monoalkylene glycol. This is suitable when a satisfactory level of conversion has been achieved in the first and second reactor units.

### Step e)

The method may optionally further comprise flowing at least a portion of the second effluent to a third reactor unit.

While the second reactor unit can convert a major portion of the unreacted alkylene oxide in the first effluent into monoalkylene glycol, some amounts of unreacted alkylene oxide may still remain in the second effluent. The third reactor unit can advantageously convert any such unreacted alkylene oxide in the second effluent into monoalkylene glycol.

As described before, when an overhead stream comprising unreacted water and unreacted alkylene oxide and a bottoms stream comprising unreacted water and monoalkylene glycol are formed in the second reactor unit, part or all of the bottoms stream is fed as the second effluent to the third reactor unit.

The second reactor unit may comprise an outlet which is in fluid communication with an inlet of the third reactor unit such that the second effluent flows from the second reactor unit to the third reactor unit.

### Step f)

Following the optional step e), the method may optionally further comprise subjecting said portion of the second effluent, in the third reactor unit, to third reaction conditions.

The third reaction conditions in the third reactor unit are non-catalytic reaction conditions. Thus, the third reactor unit comprises substantially no catalyst or comprises no catalyst.

Preferably, the third reactor unit comprises a plug flow reactor adapted to provide the third reaction conditions therein.

Preferably, substantially all or all of the unreacted alkylene oxide in said portion of the second effluent is converted into monoalkylene glycol. Preferably, the amount of alkylene oxide in the third effluent with respect to the amount of alkylene oxide in said portion of the second effluent fed to the third reactor is at most 10 wt%, at most 5.0 wt%, at most 2.0 wt%, at most 1.0 wt%, at most 0.5 wt%, at least 0.1 wt% or 0.0 wt%.

Preferably, the third effluent comprises substantially no alkylene oxide or comprises no alkylene oxide. Preferably, the amount of alkylene oxide in the third effluent with respect to the total weight of the third effluent is at most 2.0 wt%, at most 1.0 wt%, at most 0.5 wt%, at least 0.1 wt% or 0.0 wt%.

Preferably, the amount of monoalkylene glycol in the third effluent with respect to the total weight of the third effluent is 10 to 25 wt%.

Preferably, the amount of alkylene oxide in the third effluent with respect to the amount of monoalkylene glycol in the third effluent is at most 10 wt%, at most 5.0 wt%, at most 2.0 wt%, at most 1.0 wt%, at most 0.5 wt%, at least 0.1 wt% or 0.0 wt%.

Preferably, the amount of monoalkylene glycol in the third effluent with respect to the total amount of monoalkylene glycol, dialkylene glycol and trialkylene glycol in the third effluent is at least 80.0 wt%, preferably at least 85.0 wt%, more preferably at least 90.0 wt%, more preferably 91.5 wt%, more preferably at least 92.0 wt%, more preferably at least 92.5 wt%, more preferably at least 93.0 wt%, more preferably at least 93.5 wt%, more preferably at least 94.0 wt%, more preferably at least 94.5 wt%.

It is noted that the invention relates to the subject-matter defined in the independent claims alone or in combination with any possible combinations of features described herein, preferred in particular are those combinations of features that are present in the claims. It will therefore be appreciated that all combinations of features relating to the composition according to the invention; all combinations of features relating to the process according to the invention and all combinations of features relating to the composition according to the invention and features relating to the process according to the invention are described herein.

It is further noted that the term 'comprising' does not exclude the presence of other elements. However, it is also to be understood that a description on a product/composition comprising certain components also discloses a product/composition consisting of these components. The product/composition consisting of these components may be advantageous in that it offers a simpler, more economical process for the preparation of the product/composition. Similarly, it is also to be understood that a description on a process comprising certain steps also discloses a process consisting of these steps. The process consisting of these steps may be advantageous in that it offers a simpler, more economical process.

When values are mentioned for a lower limit and an upper limit for a parameter, ranges made by the combinations of the values of the lower limit and the values of the upper limit are also understood to be disclosed.

The invention is now elucidated by referring to drawings in which:

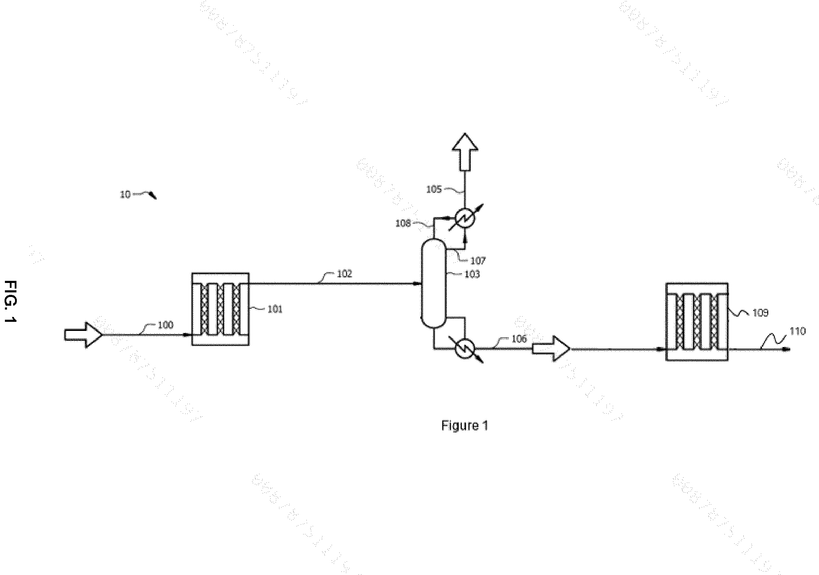

Figure 1 shows a schematic diagram of a system for carrying out an embodiment of the method of the invention.

Figure 1 shows a system 10 comprising first reactor unit 101 configured to receive feed stream 100 comprising primarily water and ethylene oxide. First reactor unit 101 is a plug flow reactor adapted to subject water and ethylene oxide to first reaction conditions sufficient to produce monoethylene glycol. Reactor 101 contains substantially no catalyst or no catalyst. Thus, the first reaction conditions are non-catalytic reaction conditions.

Reactor 101 is further configured to release first effluent 102 therefrom. First effluent 102 comprises unreacted water, unreacted ethylene oxide and monoethylene glycol and may further comprise diethylene glycol, triethylene glycol and/or polyethylene glycol.

Reactor 101 may comprise a heating mechanism adapted to heat water and ethylene oxide to a reaction temperature sufficient to produce ethylene glycol. The heating mechanism may include a heat exchanger located upstream to an inlet of reactor 101.

An outlet of reactor 101 is in fluid communication with an inlet of a second reactor unit comprising reactive distillation column 103 such that first effluent 102 flows from reactor 101 to reactive distillation column 103.

Reactive distillation column 103 comprises a catalyst bed and is adapted to provide reaction conditions so that unreacted ethylene oxide is converted to ethylene glycol under catalytic reaction conditions.

Reactive distillation column 103 is adapted to provide overhead stream 107, part of which is fed back to the column as reflux 108, and part of which is some non-condensable gases, if any (stream 105), and bottoms stream 106 as second effluent.

Overhead stream 107 may comprise unreacted water and unreacted ethylene oxide. Bottoms stream 106 comprises unreacted water and monoethylene glycol. Bottoms stream 106 may further comprise diethylene glycol, triethylene glycol and/or polyethylene glycol.

An outlet of reactive distillation column 103 is in fluid communication with an inlet of a third reactor unit 109 such that second effluent 106 flows from reactive distillation column 103 to third reactor unit 109.

Third reactor unit 109 is a plug flow reactor adapted to subject unreacted water and unreacted ethylene oxide to third reaction conditions sufficient to produce monoethylene glycol. Reactor 109 contains substantially no catalyst or no catalyst. Thus, the third reaction conditions are non-catalytic reaction conditions.

Reactor 109 is further configured to release third effluent 110 therefrom. Third effluent 110 comprises monoethylene glycol and may further comprise diethylene glycol, triethylene glycol and/or polyethylene glycol. Third effluent 110 comprises substantially no unreacted ethylene oxide

### Simulations on Ethylene Glycol Production

Simulations of ethylene glycol production using systems shown in Table 1 were run in Aspen PLUS^{™} platform. CEx 1 and CEx 2 use a system in which only a plug flow reactor is used. CEx 3 and CEx 4 use a system shown in Figure 1 wherein the catalytic reactive distillation column is replaced by a non-catalytic reactive distillation column. Ex 5 and Ex 6 use the system of the present invention shown in Figure 1. Ex 7 uses the system of the present invention shown in Figure 1 wherein the catalytic reactive distillation column is replaced by a catalytic fixed bed reactor. Feed to the first reactor unit contains only ethylene oxide (EO) and water.

**Table 1**

| | CEx 1 | CEx 2 | CEx 3 | CEx 4 | Ex 5 | Ex 6 | Ex 7 |
|---|---|---|---|---|---|---|---|
| | PFR | PFR | PFR + Non-Catalytic RD | PFR + Non-Catalytic RD | PFR + Catalytic RD | PFR + Catalytic RD | PFR + CBR |
| Products | Hourly (MT per hr) | Hourly (MT per hr) | Hourly (MT per hr) | Hourly (MT per hr) | Hourly (MT per hr) | Hourly (MT per hr) | Hourly (MT per hr) |
| Feed | | | | | | | |
| EO | 69.2 | 69.2 | 69.2 | 69.2 | 69.2 | 69.2 | 69.2 |
| WATER | 623.2 | 340.0 | 623.2 | 340 | 623.2 | 340.0 | 340.0 |
| Final stream | | | | | | | |
| MEG | 87.5 | 80.4 | 88.8 | 84.6 | 91.9 | 90.3 | 88.1 |
| DEG | 8.1 | 13.3 | 7.2 | 10.3 | 4.7 | 6.0 | 7.5 |
| TEG | 0.4 | 1.3 | 0.3 | 0.7 | 0.2 | 0.3 | 0.5 |
| Water/EO ratio (by mol) in feed | 22:1 | 12:1 | 22:1 | 12:1 | 22:1 | 12:1 | 12:1 |
| MEG selectivity in final stream (wt%) | 91.1 | 84.7 | 92.2 | 88.4 | 95.0 | 93.6 | 91.6 |

| | | | | | | | |
|---|---|---|---|---|---|---|---|
| PFR: Plug Flow Reactor RD: Reactive Distillation Column CBR: Catalytic Fixed Bed Reactor MEG Selectivity (wt%) is the amount of MEG with respect to the total of MEG, DEG and TEG. | | | | | | | |

It can be understood that the use of catalytic reactor following a plug flow reactor results in a higher MEG selectivity at the same water/ethylene oxide molar ratio (CEx 1 and CEx 3 vs Ex 5; CEx 2 and CEx 4 vs Ex 6 and Ex 7).

The high MEG selectivity can be achieved even at a lower water/ethylene oxide molar ratio, in particular the catalytic reactive distillation column achieves a very high MEG selectivity (CEx 2 and CEx 4 vs Ex 6 vs Ex 7).

Table 2 shows the compositions of the various streams in the system of Figure 1 in Ex 5.

**Table 2**

| Parameter | Unit | Feed 100 | First stream 102 | Second stream 106 | Third stream 110 |
|---|---|---|---|---|---|
| Temperature | °C | 60.0 | 159.9 | 201.2 | 201.2 |
| Pressure | bar | 22.0 | 22.0 | 15.2 | 15.2 |
| Mass Flows | tonne/hr | 692.5 | 692.5 | 692.4 | 692.4 |
| WATER | tonne/hr | 623.2 | 609.8 | 595.7 | 595.7 |
| EO | tonne/hr | 69.2 | 35.5 | 0.0 | 0.0 |
| MEG | tonne/hr | 0.0 | 45.0 | 91.9 | 91.9 |
| DEG | tonne/hr | 0.0 | 2.1 | 4.7 | 4.7 |
| TEG | tonne/hr | 0.0 | 0.1 | 0.2 | 0.2 |
| Mass Fractions | | | | | |
| WATER | | 0.90 | 0.89 | 0.86 | 0.86 |
| EO | | 0.10 | 0.05 | 0.00 | 0.0 |
| MEG | | 0.00 | 0.06 | 0.13 | 0.13 |

Table 3 shows the compositions of the various streams in the system of Figure 1 in Ex 6.

**Table 3**

| Parameter | Unit | Feed 100 | First stream 102 | Second stream 106 | Third stream 110 |
|---|---|---|---|---|---|
| Temperature | C | 60.0 | 165.9 | 203.0 | 203.0 |
| Pressure | bar | 22.0 | 22.0 | 15.2 | 15.2 |
| Mass Flows | tonne/hr | 409.2 | 409.2 | 409.2 | 409.2 |
| WATER | tonne/hr | 340.0 | 330.4 | 312.7 | 312.7 |
| EO | tonne/hr | 69.3 | 44.9 | 0.0 | 0.0 |
| MEG | tonne/hr | 0.0 | 31.9 | 90.3 | 90.3 |
| DEG | tonne/hr | 0.0 | 2.0 | 6.0 | 6.0 |
| TEG | tonne/hr | 0.0 | 0.1 | 0.3 | 0.3 |
| Mass Fractions | | | | | |
| WATER | | 0.83 | 0.81 | 0.76 | 0.76 |
| EO | | 0.17 | 0.11 | 0.00 | 0.00 |
| MEG | | 0.00 | 0.08 | 0.22 | 0.22 |

Table 4 shows the compositions of the various streams in the system of Figure 1 in Ex 7 wherein the reactive distillation column 103 is replaced by a catalytic fixed bed reactor.

**Table 4**

| Parameter | Unit | Feed 100 | First stream 102 | Second stream 106 | Third stream 110 |
|---|---|---|---|---|---|
| Temperature | C | 60.0 | 160.7 | 221.8 | 221.8 |
| Pressure | bar | 22.0 | 22.0 | 21.0 | 15.2 |
| Mass Flows | tonne/hr | 409.2 | 409.2 | 409.2 | 409.2 |
| WATER | tonne/hr | 340.0 | 331.9 | 313.0 | 313.0 |
| EO | tonne/hr | 69.3 | 49.1 | 0.0 | 0.0 |
| MEG | tonne/hr | 0.0 | 26.8 | 88.1 | 88.1 |
| DEG | tonne/hr | 0.0 | 1.4 | 7.5 | 7.5 |
| TEG | tonne/hr | 0.00 | 0.04 | 0.50 | 0.50 |
| Mass Fractions | | | | | |
| WATER | | 0.83 | 0.81 | 0.76 | 0.76 |
| EO | | 0.17 | 0.12 | 0.00 | 0.00 |
| MEG | | 0.0 | 0.07 | 0.22 | 0.22 |

## Claims

1. A method for producing monoalkylene glycol, the method comprising:
a) flowing a feed stream comprising alkylene oxide and water into a first reactor unit;
b) subjecting the feed stream, in the first reactor unit, to first reaction conditions to obtain a first effluent comprising monoalkylene glycol, unreacted alkylene oxide and unreacted water,
wherein the first reaction conditions in the first reactor unit are non-catalytic reaction conditions;
c) flowing at least a portion of the first effluent to a second reactor unit; and
d) subjecting said portion of the first effluent, in the second reactor unit, to second reaction conditions to obtain a second effluent comprising monoalkylene glycol, wherein the second reactor unit comprises a reactor comprising a catalyst bed and the second reaction conditions in the second reactor unit are catalytic reaction conditions.

2. The method of according to claim 1, wherein the reactor comprising the catalyst bed is a reactive distillation column or a catalytic fixed bed reactor, preferably the reactor comprising the catalyst bed is a reactive distillation column.

3. The method according to any of one of the preceding claims, wherein the method further comprises
e) flowing at least a portion of the second effluent to a third reactor unit and
f) subjecting said portion of the second effluent, in the third reactor unit, to third reaction conditions, wherein the third reaction conditions in the third reactor unit are non-catalytic reaction conditions.

4. The method of according to claim 3, wherein the third reactor unit comprises a plug flow reactor adapted to provide the third reaction conditions therein.

5. The method according to any one the preceding claims, wherein
step d) comprises obtaining the second effluent and an overhead stream, wherein the second effluent comprises a bottoms stream comprising unreacted water and monoalkylene glycol and the overhead comprises unreacted water and unreacted alkylene oxide and
wherein at least a portion of the overhead stream is recycled back to the reactor comprising the catalyst bed as reflux.

6. The method according to claim 5, wherein the bottoms stream further comprises dialkylene glycol and/or trialkylene glycol.

7. The method according to claim 3 or 4, wherein
step d) comprises obtaining the second effluent and an overhead stream, wherein the second effluent comprises a bottoms stream comprising unreacted water and monoalkylene glycol and the overhead comprises unreacted water and unreacted alkylene oxide,
wherein at least a portion of the overhead stream is recycled back to the reactor comprising the catalyst bed as reflux,
wherein step e) comprises flowing the bottoms stream to the third reactor unit.

8. The method according to claim 7, wherein the bottoms stream further comprises dialkylene glycol and/or trialkylene glycol.

9. The method according to any of one of the preceding claims, wherein the monoalkylene glycol is monoethylene glycol and the alkylene oxide is ethylene oxide.

10. The method according to any one of the preceding claims, wherein the first reactor unit comprises a plug flow reactor adapted to provide the first reaction conditions therein.

11. The method according to any one of the preceding claims, wherein the catalyst bed comprises a catalyst selected from the group consisting of molecular sieves such as zeolites; niobium oxide and supported niobium oxide; metals belonging to Group 1 or 2 on a support or a supported niobium; niobic acid; hydrotalcite and hydrotalcite modified with metals; heteropolyacids; ion-exchange resins; heterogenized metal complexes, such as Co/Salen on silica or polymer supports; solid superacids or superbases; and mesoporous molecular sieves in acidic or basic form with supported or exchanged metals; and combinations thereof.

12. The method of any one of the preceding claims, wherein the molar ratio of water and ethylene oxide in the feed stream is 5:1 to 30:1.

13. The method of any one of the preceding claims, wherein the molar ratio of water and ethylene oxide in the feed stream is 5:1 to 15:1.

14. The method of any one of the preceding claims, wherein the second reaction conditions in the second reactor unit comprise a reactor inlet temperature in a range of 80 to 220 °C, for example 160 to 220 °C, and a reaction pressure of 10 to 30 bar, for example 10 to 20 bar.

15. The method of any one of the preceding claims, wherein the amount of monoalkylene glycol in the second effluent with respect to the total amount of monoalkylene glycol, dialkylene glycol and trialkylene glycol in the second effluent is at least 80.0 wt%, preferably at least 85.0 wt%, more preferably at least 90.0 wt%, more preferably 91.5 wt%, more preferably at least 92.0 wt%, more preferably at least 92.5 wt%, more preferably at least 93.0 wt%, more preferably at least 93.5 wt%, more preferably at least 94.0 wt%, more preferably at least 94.5 wt%.
